# EUROPEAN PATENT APPLICATION

(11) **EP 4 141 885 A1**
(43) Date of publication of application: **01.03.2023**
(21) Application number: 22191794.1
(22) Date of filing: 23.08.2022
(51) Int. Cl.: G16H 50/20, G06N 3/02, G06N 20/00, G16H 50/70, G16H 10/20, A61B 5/00

(54) **MEDICAL INFORMATION PROCESSING SYSTEM, MEDICAL INFORMATION PROCESSING METHOD, AND PROGRAM**

(30) Priority: 25.08.2021 JP 2021137188; 22.08.2022 JP 2022132046
(71) Applicant: Canon Medical Systems Corporation, Tochigi (JP)
(72) Inventor: SAKAGUCHI, Takuya, Tochigi (JP)
(74) Representative: Marks & Clerk LLP

(57) **Abstract**

A medical information processing system of an embodiment includes an acquisition unit, an estimation unit, and an output control unit. The acquisition unit acquires medical examination data of a diagnosis subject and reply data of a medical examination by interview for the diagnosis subject. The estimation unit estimates a disease or symptoms of the diagnosis subject by inputting the examination data of the diagnosis subject to a trained model. The trained model is a model trained on the basis of a training data set in which diseases or symptoms that a learning subject suffers from are associated with examination data of the learning subject as correct answer labels. The output control unit outputs a result of comparison between estimated data based on an estimation result of the disease or symptoms of the diagnosis subject and the reply data via an output unit.

## Description

### FIELD

Embodiments disclosed in the present description and drawings relate to a medical information processing system, a medical information processing method, and a program.

### BACKGROUND

The replies of a patient in a medical examination by interview are important information for estimating the condition of the patient. The replies of a patient are unstable information that often fluctuates due to the patient's own sensitivity and feelings. Currently, medical staff assimilate and understand these fluctuations and use the fluctuations for medical treatment. Meanwhile, automation of medical treatment using artificial intelligence (AI) is under review. However, uncertainty may be a problem in medical treatment using AI.

### SUMMARY

An object of embodiments disclosed in the present description and drawings is to treat a patient while taking the uncertainty of AI into account. However, the object of the embodiments disclosed in the present description and drawings is not limited to the above object. An object corresponding to each effect according to each configuration represented in embodiments which will be described later can be positioned as another object.
(1) A medical information processing system of an embodiment includes an acquisition unit, an estimation unit, and an output control unit. The acquisition unit acquires medical examination data of a diagnosis subject and reply data of a medical examination by interview for the diagnosis subject. The estimation unit estimates a disease or symptoms of the diagnosis subject by inputting the examination data of the diagnosis subject to a trained model. The trained model is a model trained on the basis of a training data set in which diseases or symptoms that a learning subject suffers from are associated with examination data of the learning subject as correct answer labels. The output control unit outputs a result of comparison between estimated data based on an estimation result of the disease or symptoms of the diagnosis subject and the reply data via an output unit.
(2) The estimation unit may estimate a plurality of diseases that the diagnosis subject may suffer from or symptoms of each of the plurality of diseases that the diagnosis subject may suffer from using the model. The medical information processing system may further include a determination unit. The determination unit may determine whether or not the reply data is similar to each of a plurality of pieces of estimated data corresponding to each of the plurality of diseases that the diagnosis subject may suffer from.
(3) The determination unit may calculate a similarity between each of the plurality of pieces of estimated data and the reply data. The output control unit may preferentially output estimated data having a higher similarity to the reply data among the plurality of pieces of estimated data via the output unit.
(4) The output control unit may output information representing that the higher a similarity of the estimated data is, the higher a reliability of the model is via the output unit.
(5) The medical information processing system may further include a learning unit. The learning unit may train the model such that the estimated data is more similar to the reply data.
(6) The model may be trained on the basis of the training data set in which the diseases of the learning subject are associated with the examination data of the learning subject as correct answer labels. The estimation unit may estimate the disease of the diagnosis subject by inputting the examination data of the diagnosis subject to the model, and may further estimate symptoms of the diagnosis subject from the disease of the diagnosis subject using corresponding information in which symptoms are associated with diseases. The output control unit may output a result of comparison between the estimated data representing the estimated symptoms of the diagnosis subject and the reply data via the output unit.
(7) The model may be trained on the basis of the training data set in which symptoms of the learning subject are associated with the examination data of the learning subject as correct answer labels. The estimation unit may estimate symptoms of the diagnosis subject by inputting the examination data of the diagnosis subject to the model. The output control unit may output a result of comparison between the estimated data representing the estimated symptoms of the diagnosis subject and the reply data via the output unit.
(8) A medical information processing system including an acquisition unit, an estimation unit, and an output control unit. The acquisition unit acquires medical examination data of a diagnosis subject and reply data of a medical examination by interview for the diagnosis subject. The estimation unit estimates a disease of the diagnosis subject by inputting the examination data of the diagnosis subject to a model trained on the basis of a training data set in which diseases that a learning subject suffers from are associated with examination data of the learning subject as correct answer labels. The estimation unit further estimates symptoms of the diagnosis subject from the disease of the diagnosis subject using corresponding information in which symptoms are associated with diseases. The output control unit outputs a result of comparison between estimated data representing an estimation result of the symptoms of the diagnosis subject and the reply data via an output unit.
(9) A medical information processing system including an acquisition unit, an estimation unit, and an output control unit. The acquisition unit acquires medical examination data of a diagnosis subject and reply data of a medical examination by interview for the diagnosis subject. The estimation unit estimates symptoms of the diagnosis subject by inputting the examination data of the diagnosis subject to a model trained on the basis of a training data set in which symptoms of a learning subject are associated with examination data of the learning subject as correct answer labels. The output control unit outputs a result of comparison between estimated data representing an estimation result of the symptoms of the diagnosis subject and the reply data via an output unit.
(10) A medical information processing method using a computer, including:
   acquiring medical examination data of a diagnosis subject and reply data of a medical examination by interview for the diagnosis subject;
   estimating a disease or symptoms of the diagnosis subject by inputting the examination data of the diagnosis subject to a model trained on the basis of a training data set in which diseases or symptoms that a learning subject suffers from are associated with examination data of the learning subject as correct answer labels; and
   outputting a result of comparison between estimated data based on an estimation result of the disease or symptoms of the diagnosis subject and the reply data via an output unit.
(11) A program for causing a computer to execute:
   acquiring medical examination data of a diagnosis subject and reply data of a medical examination by interview for the diagnosis subject;
   estimating a disease or symptoms of the diagnosis subject by inputting the examination data of the diagnosis subject to a model trained on the basis of a training data set in which diseases or symptoms that a learning subject suffers from are associated with examination data of the learning subject as correct answer labels; and
   outputting a result of comparison between estimated data based on an estimation result of the disease or symptoms of the diagnosis subject and the reply data via an output unit.

### BRIEF DESCRIPTION OF THE DRAWINGS

FIG. 1 is a diagram showing a configuration example of a medical information processing system in a first embodiment.
FIG. 2 is a diagram showing a configuration example of a user interface in the first embodiment.
FIG. 3 is a diagram showing a configuration example of a medical information processing device in the first embodiment.
FIG. 4 is a flowchart showing a flow of a series of processing of a processing circuit according to the first embodiment.
FIG. 5 is a diagram for describing reply data
FIG. 6 is a diagram showing an example of an output result of a trained model according to the first embodiment.
FIG. 7 is a diagram showing an example of a display screen.
FIG. 8 is a diagram showing an example of an output result of a trained model according to a second embodiment.
FIG. 9 is a diagram showing an example of results of similarity.
FIG. 10 is a diagram for describing a retraining method of a trained model.
FIG. 11 is a diagram showing an example of an output result of a trained model according to a fourth embodiment.

### DETAILED DESCRIPTION

Hereinafter, a medical information processing system, a medical information processing method, and a program of embodiments will be described with reference to the drawings.

### (First embodiment)

### [Configuration of medical information processing system]

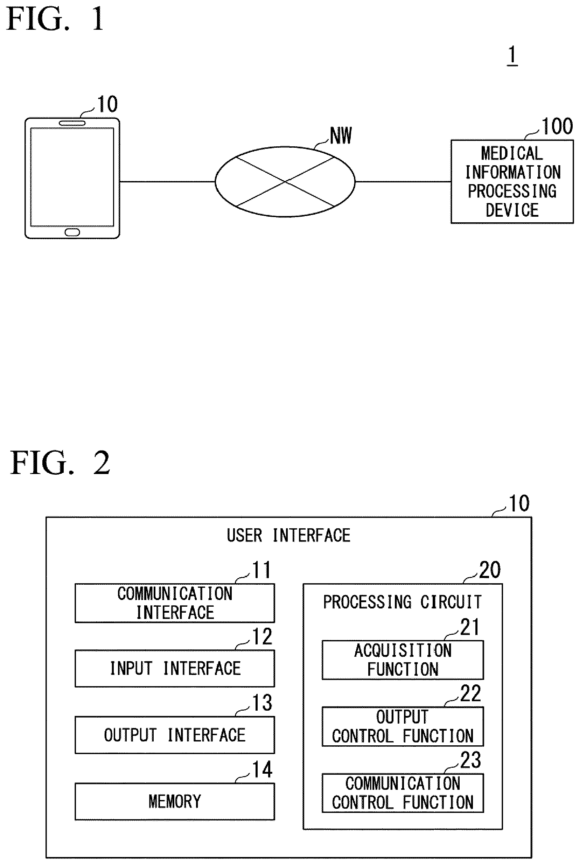

FIG. 1 is a diagram showing a configuration example of a medical information processing system 1 in a first embodiment. The medical information processing system 1 includes, for example, a user interface 10 and a medical information processing device 100. The user interface 10 and the medical information processing device 100 are connected such that they can communicate with each other via a communication network NW.

The communication network NW may mean an information communication network in general using telecommunications technology. For example, the communication network NW includes a telephone communication line network, an optical fiber communication network, a cable communication network, a satellite communication network, and the like in addition to a wireless/wired local area network (LAN) such as a hospital backbone LAN and the Internet.

The user interface 10 is used by patients and medical staff. For example, the user interface 10 is a touch interface or a voice user interface, and more specifically, a terminal device such as a personal computer, a tablet terminal, or a mobile phone. Medical staff are typically doctors but may be nurses or other people involved in medical treatment. For example, a patient inputs his/her own replies in a medical examination by interview to the user interface 10 through touch or voice input. A medical staff member may perform an oral medical examination by interview for the patient, listens to replies from the patient, and input listening comprehension results to the user interface 10.

In the present embodiment, "medical treatment" may include not only treatment such as surgery and medication but also examination up to treatment or after treatment and any other medical practices up to or after the treatment.

The user interface 10 transmits information input by a patient or a medical staff member to the medical information processing device 100 via the communication network NW or receives information from the medical information processing device 100.

The medical information processing device 100 receives information from the user interface 10 via the communication network NW and processes the received information. Then, the medical information processing device 100 transmits the processed information to the user interface 10 via the communication network NW. In addition to or instead of transmitting the processed information to the user interface 10, the medical information processing device 100 may transmit the processed information to a dedicated terminal of a medical staff member installed in a hospital.

The medical information processing device 100 may be a single device or may be a system in which a plurality of devices connected via the communication network NW operate in cooperation. That is, the medical information processing device 100 may be realized by a plurality of computers (processors) included in a distributed computing system or a cloud computing system. The medical information processing device 100 does not necessarily have to be a separate device from the user interface 10 and may be a device integrated with the user interface 10.

### [Configuration of terminal device]

FIG. 2 is a diagram showing a configuration example of the user interface 10 in the first embodiment. The user interface 10 includes, for example, a communication interface 11, an input interface 12, an output interface 13, a memory 14, and a processing circuit 20.

The communication interface 11 communicates with the medical information processing device 100 or the like via the communication network NW. The communication interface 11 includes, for example, a network interface card (NIC), an antenna for wireless communication, and the like.

The input interface 12 receives various input operations from an operator (for example, a patient), converts the received input operations into electrical signals and outputs the electrical signals to the processing circuit 20. For example, the input interface 12 includes a mouse, a keyboard, a trackball, a switch, a button, a joystick, a touch panel, and the like. The input interface 12 may be, for example, a user interface that receives voice input, such as a microphone. When the input interface 12 is a touch panel, the input interface 12 may also have a display function of a display 13a included in the output interface 13 which will be described later.

In the present description, the input interface 12 is not limited to one including physical operation parts such as a mouse and a keyboard. For example, examples of the input interface 12 include an electrical signal processing circuit that receives an electrical signal corresponding to an input operation from an external input device provided separately from the device and outputs the electrical signal to a control circuit.

The output interface 13 includes, for example, the display 13a, a speaker 13b, and the like.

The display 13a displays various types of information. For example, the display 13a displays an image generated by the processing circuit 20, a graphical user interface (GUI) for receiving various input operations from an operator, and the like. For example, the display 13a is a liquid crystal display (LCD), a cathode ray tube (CRT) display, an organic electroluminescence (EL) display, or the like.

The speaker 13b outputs information input from the processing circuit 20 as voice information.

The memory 14 is realized by, for example, a semiconductor memory element such as a random access memory (RAM) or a flash memory, a hard disk, or an optical disc. These non-transient storage media may be realized by other storage devices connected via the communication network NW, such as a network attached storage (NAS) and an external storage server device. The memory 14 may include a non-transient storage medium such as a read only memory (ROM) or a register.

The processing circuit 20 includes, for example, an acquisition function 21, an output control function 22, and a communication control function 23. The processing circuit 20 realizes these functions by, for example, a hardware processor (computer) executing a program stored in the memory 14 (storage circuit).

The hardware processor in the processing circuit 20 means, for example, a circuit (circuitry) such as a central processing unit (CPU), a graphics processing unit (GPU), an application specific integrated circuit (ASIC), or a programmable logic device (for example, a simple programmable logic device (SPLD) or a complex programmable logic device (CPLD), or a field programmable gate array (FPGA)). Instead of storing the program in the memory 14, the program may be configured to be directly embedded in the circuit of the hardware processor. In this case, the hardware processor realizes the functions by reading and executing the program embedded in the circuit. The aforementioned program may be stored in the memory 14 in advance or may be stored in a non-temporary storage medium such as a DVD or a CD-ROM and installed in the memory 14 from the non-temporary storage medium when the non-temporary storage medium is set in a drive device (not shown) of the user interface 10. The hardware processor is not limited to one configured as a single circuit and may be configured as one hardware processor by combining a plurality of independent circuits to realize each function. A plurality of components may be integrated into one hardware processor to realize each function.

The acquisition function 21 acquires input information via the input interface 12 or acquires information from the medical information processing device 100 via the communication interface 11.

The output control function 22 displays information acquired by the acquisition function 21 on the display 13a or outputs the information through the speaker 13b.

The communication control function 23 transmits information input to the input interface 12 to the medical information processing device 100 via the communication interface 11.

### [Configuration of medical information processing device]

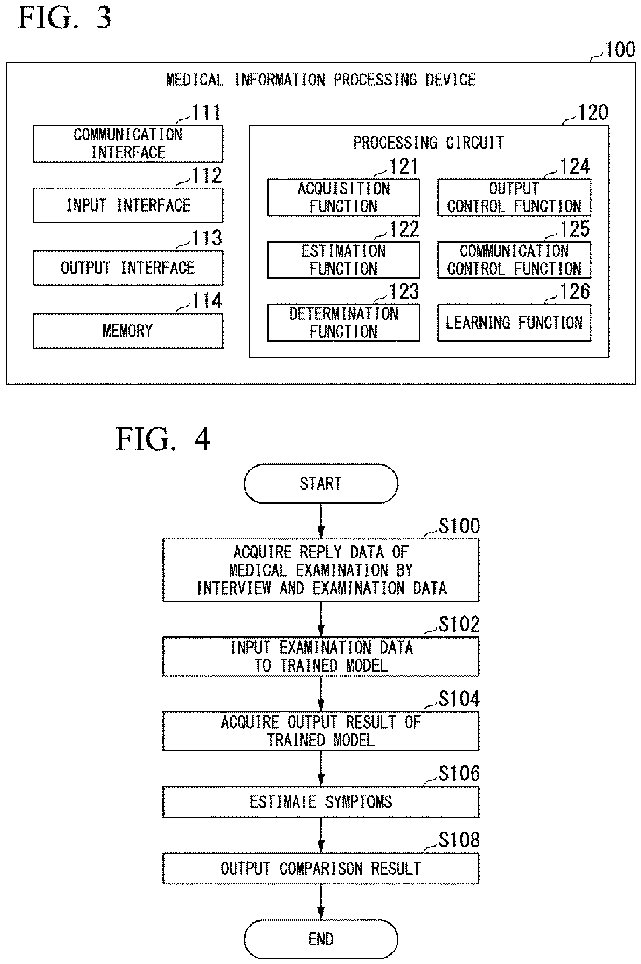

FIG. 3 is a diagram showing a configuration example of the medical information processing device 100 in the first embodiment. The medical information processing device 100 includes, for example, a communication interface 111, an input interface 112, an output interface 113, a memory 114, and a processing circuit 120.

The communication interface 111 communicates with the user interface 10 and the like via the communication network NW. The communication interface 111 includes, for example, an NIC or the like. The communication interface 111 is an example of an "output unit."

The input interface 112 receives various input operations from an operator, converts the received input operations into electrical signals and outputs the electrical signals to the processing circuit 120. For example, the input interface 112 includes a mouse, a keyboard, a trackball, a switch, a button, a joystick, a touch panel, and the like. The input interface 112 may be, for example, a user interface that receives voice input, such as a microphone. When the input interface 112 is a touch panel, the input interface 112 may also have a display function of a display 113a included in the output interface 113 which will be described later.

In the present description, the input interface 112 is not limited to one provided with physical operating parts such as a mouse and a keyboard. For example, examples of the input interface 112 include an electrical signal processing circuit that receives an electrical signal corresponding to an input operation from an external input device provided separately from the device and outputs the electrical signal to a control circuit.

The output interface 113 includes, for example, the display 113a, a speaker 113b, and the like. The output interface 113 is another example of the "output unit".

The display 113a displays various types of information. For example, the display 113a displays an image generated by the processing circuit 120, a GUI for receiving various input operations from the operator, and the like. For example, the display 113a is an LCD, a CRT display, an organic EL display, or the like.

The speaker 113b outputs information input from the processing circuit 120 as voice information.

The memory 114 is realized by, for example, a semiconductor memory element such as a RAM or a flash memory, a hard disk, or an optical disc. These non-transient storage media may be realized by other storage devices connected via a communication network NW, such as an NAS or an external storage server device. The memory 114 may include a non-transient storage medium such as a ROM or a register.

The memory 114 stores model information, a case database, and the like in addition to a program executed by a hardware processor.

The model information is information (program or data structure) that defines a trained model MDL. The trained model MDL may be implemented by a deep neural network (DNN) such as a convolutional neural network (CNN), for example. The trained model MDL is not limited to a DNN and may be implemented by other models such as a support vector machine, a decision tree, a naive Bayes classifier, and a random forest. Details of the trained model MDL will be described later.

When the trained model MDL is implemented by a DNN, the model information includes, for example, coupling information on how units included in an input layer constituting the DNN, one or more hidden layers (intermediate layers), and an output layer are coupled with each other, weight information on how many coupling coefficients are provided to data input/output between coupled units, and the like. The coupling information includes, for example, information for specifying the number of units included in each layer and the type of a unit that is a coupling destination of each unit, an activation function for realizing each unit, a gate provided between units in a hidden layer, and the like. The activation function for realizing a unit may be, for example, a rectified linear unit (ReLU) function, an exponential linear units (ELU) function, a clipping function, a Sigmoid function, a step function, a hyperbolic tangent function, an equality function, or the like. The gate selectively passes or weights data transmitted between units, for example, depending on a value (e.g., 1 or 0) returned by the activation function. The coupling coefficients include, for example, a weight applied to output data when data is output from a unit of a certain layer to a unit of a deeper layer in a hidden layer of a neural network. The coupling coefficients may include a bias component specific to each layer, and the like.

The case database is, for example, a database in which one or several symptoms (cases) are associated with each disease. For example, the disease "lung cancer" is associated with several typical symptoms such as coughing, sputum production, bloody sputum, fever, suffocation, palpitation, chest pain, chills, and malaise. The case database is an example of "correspondence information."

The processing circuit 120 includes, for example, an acquisition function 121, an estimation function 122, a determination function 123, an output control function 124, a communication control function 125, and a learning function 126. The acquisition function 121 is an example of an "acquisition unit," the estimation function 122 is an example of an "estimation unit," and the determination function 123 is an example of a "determination unit." The output control function 124 is an example of an "output control unit" and the communication control function 125 is another example of the "output control unit." The learning function 126 is an example of a "learning unit."

The processing circuit 120 realizes these functions by, for example, a hardware processor (computer) executing a program stored in the memory 114 (storage circuit).

The hardware processor in the processing circuit 120 means, for example, a circuit (circuitry) such as a CPU, a GPU, an application specific integrated circuit, or a programmable logic device (for example, a simple programmable logic device or a complex programmable logic device, or a field programmable gate array). Instead of storing the program in the memory 114, the program may be configured to be directly embedded in the circuit of the hardware processor. In this case, the hardware processor realizes the functions by reading and executing the program embedded in the circuit. The aforementioned program may be stored in the memory 114 in advance, or may be stored in a non-temporary storage medium such as a DVD or a CD-ROM and installed in the memory 114 from the non-temporary storage medium when the non-temporary storage medium is set in a drive device (not shown) of the medical information processing device 100. The hardware processor is not limited to one configured as a single circuit, and may be configured as one hardware processor by combining a plurality of independent circuits to realize each function. A plurality of components may be integrated into one hardware processor to realize each function.

### [Processing flow of medical information processing device]

Hereinafter, a series of processing performed by the processing circuit 120 of the medical information processing device 100 will be described with reference to a flowchart. FIG. 4 is a flowchart showing a flow of a series of processing of the processing circuit 120 according to the first embodiment.

First, the acquisition function 121 acquires reply data of a patient that is a diagnosis target (hereinafter, a diagnosis subject) for a medical examination by interview from the user interface 10 via the communication interface 111 and further acquires examination data of the diagnosis subject from examination equipment (step S100).

The examination equipment is equipment for medically examining patients and is, for example, an X-ray computed tomography (CT) device, a magnetic resonance imaging (MRI) device, a mammography device, a sound imaging diagnostic device, a nuclear medicine diagnostic device, a body fluid analysis device, devices for measuring vital signs, or the like. Examination data is quantitative digital data obtained by measuring biological information of the diagnosis subject by the above-mentioned various types of examination equipment. On the other hand, reply data is qualitative digital data including the subjectivity of the diagnosis subject. That is, the acquisition function 121 acquires quantitative data and qualitative data regarding the diagnosis subject.

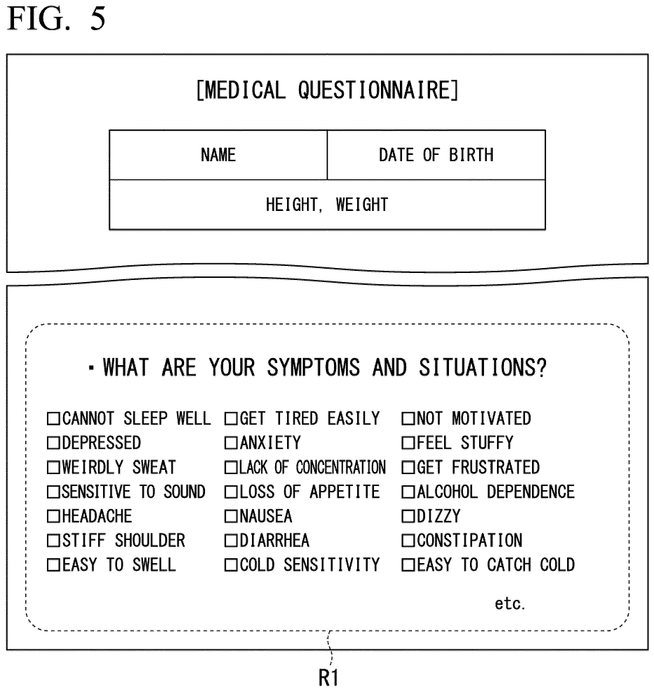

FIG. 5 is a diagram for describing reply data. For example, when a diagnosis subject visits a medical institution, he/she is required to fill necessary items (R1 in the figure) such as the current symptoms, a medical history, and presence/absence of allergies in a medical questionnaire as shown in the figure. The medical questionnaire may be displayed on the display 13a of the user interface 10 or may be printed on paper and distributed to the patient. When the patient has filled replies in the paper medical questionnaire, a medical staff member of the medical institution may input the fill-in content to the user interface 10. At this time, an optical character recognition/reader (OCR) may be used. The question content of the medical questionnaire may be output as voice from the speaker 113b of the user interface 10 or may be read aloud by a medical staff member of the medical institution. When the patient speaks replies to the user interface 10, the user interface 10 may obtain the replies spoken by the patient via a microphone. Alternatively or additionally, a medical staff member may listen to the replies spoken by the patient. When the medical staff member listens to the replies from the patient, the medical staff member may input listening comprehension results to the user interface 10. It is not necessary for questions to be determined in advance like a medical questionnaire, and a medical staff member may freely determine the contents of questions at the timing of examination. Replies to a medical examination by interview are medically called a chief complaint. Therefore, reply data may be replaced by chief complaint data.

The description of the flowchart of FIG. 4 will be returned to. When the reply data and the examination data regarding the medical examination by interview are acquired by the acquisition function 121, the estimation function 122 inputs the examination data of the diagnosis subject acquired by the acquisition function 121 to the trained model MDL defined by model information stored in the memory 141 (step S102).

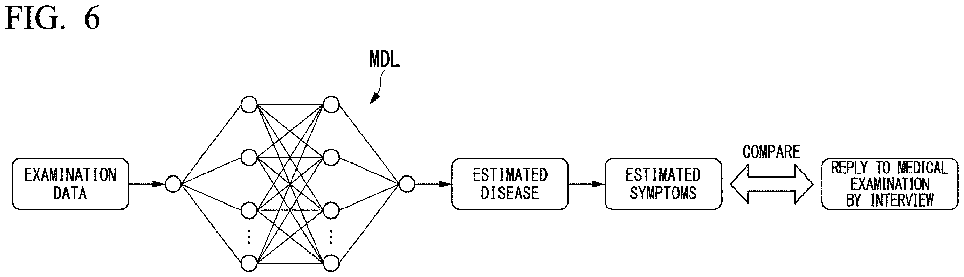

FIG. 6 is a diagram showing an example of an output result of the trained model MDL according to the first embodiment. The trained model MDL is typically a machine learning model trained using a data set in which diseases that a learning subject has already suffered from or is likely to suffer from in the future are associated with examination data of the learning subject as a correct answer label (also referred to as a target) as training data. In other words, the trained model MDL is a machine learning model trained to output diseases that a learning subject has already suffered from or is likely to suffer from in the future when examination data of the learning subject is input. The learning subject may be a patient who has been treated in the past. That is, the learning subject may be the same person as the diagnosis subject or may be a different person.

As illustrated, the trained model MDL trained using such training data outputs diseases of a certain patient as an estimation result when examination data of the patient is input.

The estimation result of the trained model MDL is represented by, for example, a multidimensional vector or tensor. The vector or tensor includes a plausibility (a probability) of being a disease as an element value. For example, it may be assumed that there are a total of three types of diseases that the diagnosis subject can suffer from: disease A, disease B, and disease C. In this case, the vector or tensor can be represented as (el, e2, e3) where the probability of disease A is e1, the probability of disease B is e2, and the probability of disease C is e3.

The description of the flowchart in FIG. 4 will be returned to. Next, the estimation function 122 acquires an estimation result from the trained model MDL to which the examination data of the diagnosis subject has been input (step S104). The estimation results output by the trained model MDL include a disease that the diagnosis subject has already suffered from or a disease that it is estimated that the diagnosis subject may suffer from in the future, and more specifically, includes a vector or tensor having the probability of being a disease as an element value.

Next, the estimation function 122 estimates the symptoms of the diagnosis subject based on the estimation result (that is, the disease of the diagnosis subject) of the trained model MDL (step S106). For example, the estimation function 122 estimates a disease corresponding to an element having a largest value among a plurality of elements included in the vector or tensor output by the trained model MDL as the disease of the diagnosis subject. Then, the estimation function 122 searches a plurality of diseases registered in the case database for the estimated disease of the diagnosis subject and estimates one or a plurality of symptoms associated with the disease of the diagnosis subject in the case database as symptoms that the diagnosis subject may develop. Data representing a symptom estimated from a disease output by the trained model MDL in response to input of examination data is an example of "estimated data."

Next, the output control function 124 outputs a result of comparison between the estimated data representing the symptoms of the diagnosis subject estimated by the estimation function 122 and the reply data of the diagnosis subject acquired by the acquisition function 121 through the output interface 113 (step S108). Accordingly, processing of this flowchart ends.

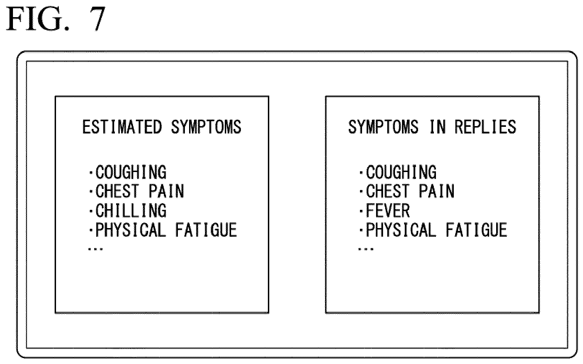

FIG. 7 is a diagram showing an example of a screen of the display 113a. As illustrated, for example, the output control function 124 may display symptoms of the diagnosis subject estimated by the estimation function 122 in comparison with symptoms in replies of the diagnosis subject on the display 113a. By performing such a display, a medical staff member can check the symptoms of the diagnosis subject estimated using the trained model MDL and the symptoms in replies of the diagnosis subject. As a result, when the symptoms in replies of the diagnosis subject are compared on the assumption that these symptoms are correct, for example, it is possible to determine whether or not the symptoms of the diagnosis subject have been estimated correctly using the trained model MDL. If the symptoms of the diagnosis subject estimated using the trained model MDL do not match the symptoms in replies of the diagnosis subject on the assumption that the symptoms in replies of the diagnosis subject are correct, the medical staff member can determine that the accuracy of the trained model MDL is not sufficient and can diagnose patients more carefully as compared to a case in which both symptoms match. If the symptoms of the diagnosis subject estimated by the estimation function 122 and the symptoms in replies of the diagnosis subject do not match on the assumption (under a precondition) that the symptoms in replies of the diagnosis subject are correct, the output control function 124 may display predetermined information on the display 113a to urge the medical staff member to diagnose the patients more carefully as compared to a case in which both symptoms match on the assumption (under the precondition) that the symptoms in replies of the diagnosis subject are correct.

The communication control function 125 may transmit estimated data and reply data to the user interface 10 via the communication interface 111. When the communication interface 11 receives the estimated data and the reply data from the medical information processing device 100, the output control function 22 of the user interface 10 may display symptoms indicated by the data (that is, estimated symptoms and symptoms in replies) on the display 13a of the output interface 13 as an image or output the symptoms as voice output through the speaker 13b.

According to the first embodiment described above, the processing circuit 120 of the medical information processing device 100 acquires examination data of a diagnosis subject and reply data in a medical examination by interview. The processing circuit 120 estimates the disease of the diagnosis subject by inputting the examination data of the diagnosis subject to the trained model MDL trained in advance. The processing circuit 120 further estimates symptoms of the diagnosis subject from the estimated disease of the diagnosis subject using the case database. Then, the processing circuit 120 outputs a result of comparison between the estimated symptoms of the diagnosis subject and the symptoms in replies of the diagnosis subject via the output interface 113. Accordingly, medical staff can treat the patient while taking the uncertainty of the trained model MDL into account.

### (Second embodiment)

Hereinafter, a second embodiment will be described. The second embodiment differs from the first embodiment in that symptoms of each of a plurality of diseases that a diagnosis subject may suffer from are estimated. Hereinafter, differences from the first embodiment will mainly be described, and similarities shared with the first embodiment will be omitted. In the description of the second embodiment, parts the same as those of the first embodiment will be denoted with the same reference numerals.

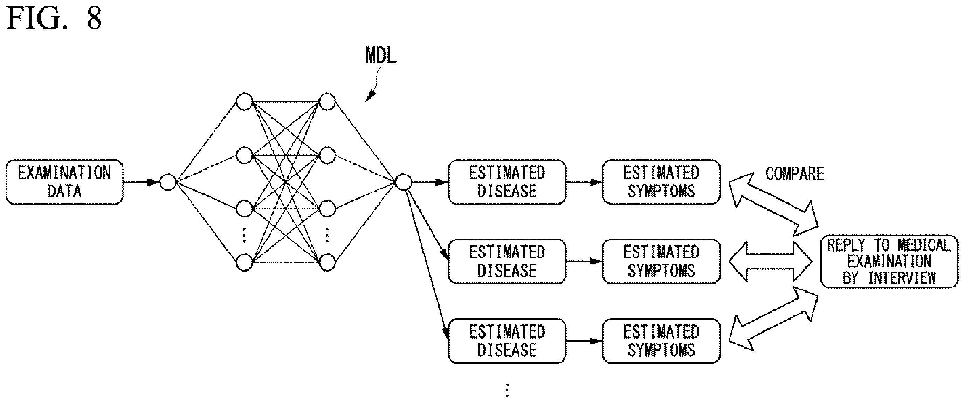

FIG. 8 is a diagram showing an example of an output result of a trained model MDL according to the second embodiment. The estimation function 122 according to the second embodiment estimates a disease corresponding to each of a plurality of elements included in a vector or a tensor output by the trained model MDL as a disease of a diagnosis subject. As described above, for example, the vector or the tensor is represented as (e1, e2, e3). In this case, the estimation function 122 estimates a total of three types of diseases: disease A corresponding to the element e1, disease B corresponding to element e2, and disease C corresponding to the element e3 as diseases of the diagnosis subject.

When the estimation function 122 estimates a plurality of diseases of the diagnosis subject, the estimation function 122 estimates symptoms of each disease using the case database as shown in FIG. 8. For example, when the estimation function 122 estimates disease A, disease B, and disease C, the estimation function 122 estimates one or more symptoms associated with disease A, one or more symptoms associated with disease B, and one or more symptoms associated with disease C as symptoms of the diagnosis subject with reference to the case database.

When a plurality of diseases are estimated and symptoms are estimated for each disease, the determination function 123 determines whether or not symptoms in replies of the diagnosis subject (that is, reply data) are similar to the symptoms estimated for each disease (that is, estimated data).

For example, it is assumed that the diagnosis subject reports four symptoms such as "coughing," "chest pain," "chill," and "physical fatigue,", whereas the estimation function 122 estimates a disease of the diagnosis subject as "lung cancer" and estimates four symptoms such as "coughing," "chest pain," "fever," and "general malaise" as symptoms of "lung cancer." The determination function 123 calculates a similarity between reply data and estimated data depending on a matching ratio of these symptoms. Specifically, the similarity may be calculated as 1 if all 4 cases match, the similarity may be calculated as 0.75 if 3 of the 4 cases match, the similarity may be calculated as 0.5 if 2 of the 4 cases match, the similarity may be calculated as 0.25 if one of the 4 cases matches, and the similarity may be calculated as 0.0 if none of the 4 cases matches.

The output control function 124 preferentially outputs a disease having symptoms having a high similarity to the symptoms in replies of the diagnosis subject among the plurality of diseases estimated by the estimation function 122 via the output interface 113.

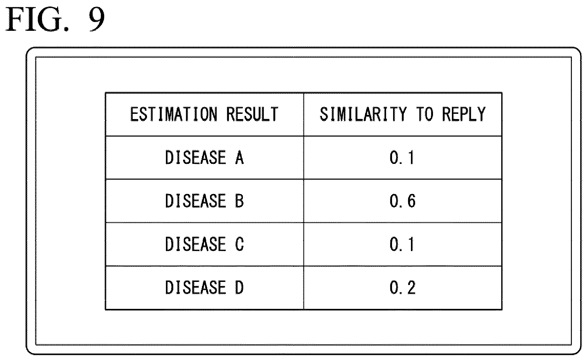

FIG. 9 is a diagram showing an example of results of similarity. In the illustrated example, the similarity of the symptoms of disease A to the symptoms in replies of the diagnosis subject is 0.1, the similarity of the symptoms of disease B to the symptoms in replies of the diagnosis subject is 0.6, the similarity of the symptoms of disease C to the symptoms in replies of the diagnosis subject is 0.1, and the similarity of the symptoms of disease D to the symptoms in replies of the diagnosis subject is 0.2. In such a case, the output control function 124 may output disease B having the highest similarity among the four symptoms with the highest priority, output disease D having the next highest similarity, and finally output diseases A and C having the lowest similarity. Specifically, the output control function 124 may display the diseases in sequential order from a disease having a highest similarity. In this case, the display 113a displays the diseases in the order of B, D, A, C from the top in a ranking format, for example.

At this time, the output control function 124 may output only diseases having a similarity equal to or higher than a threshold value. For example, when the threshold value is 0.5, the output control function 124 may output only disease B. The output control function 124 may output a predetermined number of diseases having a high similarity. For example, when the predetermined number is 2, the output control function 124 may output only diseases B and D. This can reduce consumption of computer resources.

Further, the output control function 124 may output information representing that the higher the similarity of the disease to the symptoms in replies of the diagnosis subject among the plurality of diseases estimated by the estimation function 122, the higher the reliability of the trained model MDL via the output interface 113. Accordingly, medical staff can consider diagnosis results so as to increase the reliability, and if the reliability is high, can determine that the trained model MDL has high certainty.

According to the second embodiment described above, the processing circuit 120 estimates symptoms of each of a plurality of diseases that a diagnosis subject can suffer from. The processing circuit 120 calculates a similarity between the symptoms for each disease and symptoms in replies of the diagnosis subject. Then, the processing circuit 120 preferentially outputs a disease having symptoms having a high similarity to the symptoms in replies of the diagnosis subject among the plurality of diseases that the diagnosis subject can suffer from. Accordingly, medical staff can be preferentially informed of, for example, a disease estimated to develop symptoms with a high similarity even if the disease has low certainty among the plurality of diseases estimated by the trained model MDL. As a result, the medical staff can treat the patient while better taking the uncertainty of the trained model MDL into account.

### (Third embodiment)

Hereinafter, a third embodiment will be described. The third embodiment differs from the above-described embodiments in that estimated symptoms of a diagnosis subject are compared with symptoms in replies of the diagnosis subject and the trained model MDL is retrained on the basis of a comparison result. Hereinafter, differences from the first embodiment and the second embodiment will mainly be described, and similarities shared with the first embodiment and the second embodiment will be omitted. In the description of the third embodiment, parts the same as those of the first embodiment and the second embodiment will be denoted with the same reference numerals.

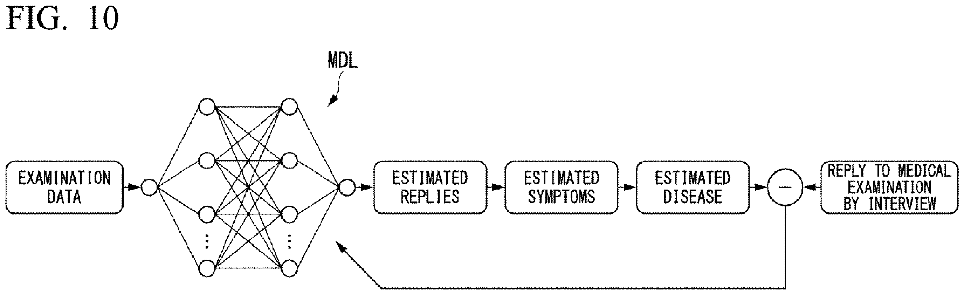

FIG. 10 is a diagram for describing a retraining method of the trained model MDL. When the estimation function 122 estimates a disease of a diagnosis subject from examination data of the diagnosis subject using the trained model MDL and estimates symptoms from the disease of the diagnosis subject using the case database, the estimation function 122 further estimates replies (in other words, chief complaint) to a medical examination by interview which would be reported by patients who developed the symptoms from the symptoms of the diagnosis subject. For example, the estimation function 122 may estimate replies to a medical examination by interview from the symptoms of the diagnosis subject using a technique such as natural language processing.

The learning function 126 adjusts parameters (weighting coefficients, a bias component, and the like) of the trained model MDL such that the replies to the medical examination by interview estimated by the estimation function 122 are more similar to replies of the diagnosis subject in a medical examination by interview. For example, the learning function 126 calculates a difference between the replies of the diagnosis subject in the medical examination by interview and the replies to the medical examination by interview estimated by the estimation function 122 and sets the parameters of the trained model MDL such that the calculated difference becomes zero. Accordingly, the uncertainty of the trained model MDL can be reduced.

According to the third embodiment described above, the processing circuit 120 retrains the trained model MDL such that estimated symptoms of the diagnosis subject are more similar to symptoms in replies of the diagnosis subject. Accordingly, it is possible to reduce the uncertainty of the trained model MDL.

### (Fourth embodiment)

Hereinafter, a fourth embodiment will be described. The fourth embodiment differs from the above-described embodiments in that the trained model MDL outputs symptoms. Hereinafter, differences from the first to third embodiments will mainly be described, and similarities shared with the first to third embodiments will be omitted. In the description of the fourth embodiment, parts the same as those of the first to third embodiments will be denoted with the same reference numerals.

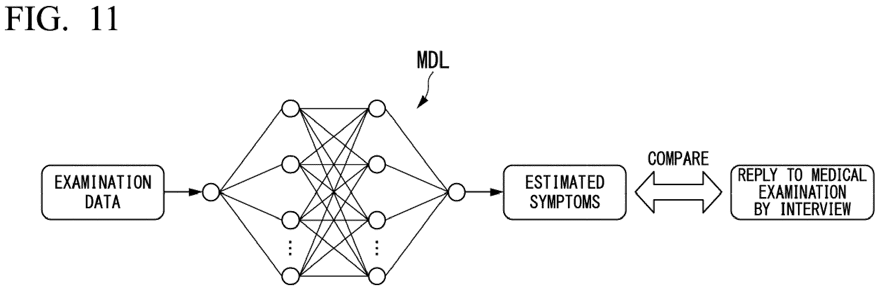

FIG. 11 is a diagram showing an example of an output result of the trained model MDL according to the fourth embodiment. Training data in the fourth embodiment may be a data set in which a typical symptom of a disease that a learning subject has already suffered from or a typical symptom of a disease that the subject is likely to suffer from in the future is associated with examination data of the learning subject as a correct answer label (also referred to as a target). The trained model MDL trained using such training data outputs symptoms that can be developed by a diagnosis subject when the examination data of the diagnosis subject is input thereto. Data representing the symptoms output by the trained model MDL in response to input of the examination data is another example of "estimated data."

An estimation result (estimated data) of the trained model MDL is represented by, for example, a multidimensional vector or a tensor, as in the above-described embodiments. The vector or tensor includes the plausibility (probability) of being a symptom as an element value. For example, it is assumed that there are a total of three types of symptoms of diseases that the diagnosis subject can suffer from: symptom X, symptom Y, and symptom Z. In this case, the vector or tensor can be represented as (el, e2, e3) where the probability of symptom X is e1, the probability of symptom Y is e2, and the probability of symptom Z is e3.

The output control function 124 outputs a result of comparison between estimated data representing symptoms of the diagnosis subject estimated by the estimation function 122 and reply data of the diagnosis subject acquired by the acquisition function 121 via the output interface 113.

According to the fourth embodiment described above, the processing circuit 120 of the medical information processing device 100 acquires examination data of the diagnosis subject and reply data to a medical examination by interview. The processing circuit 120 estimates symptoms of the diagnosis subject by inputting the examination data of the diagnosis subject to the trained model MDL trained in advance. Then, the processing circuit 120 outputs a result of comparison between the estimated symptoms of the diagnosis subject and symptoms in replies of the diagnosis subject via the output interface 113. Accordingly, medical staff can treat the patient while taking the uncertainty of the trained model MDL into account.

### (Fifth embodiment)

Hereinafter, a fifth embodiment will be described. The fifth embodiment differs from the above-described embodiments in that an output result of the trained model MDL is weighted or biased on the basis of the age of a diagnosis subject, a period elapsed since the diagnosis subject suffered from a disease (onset period), the experience of replying of the diagnosis subject in a medical examination by interview, and the like. Hereinafter, differences from the first to fourth embodiments will mainly be described, and similarities shared with the first to fourth embodiments will be omitted. In description of the fifth embodiment, parts the same as those of the first to fourth embodiments will be denoted with the same reference numerals.

For example, children and the elderly are more likely to have fluctuations in replying to medical examinations by interview than adults. Therefore, the estimation function 122 in the fifth embodiment may apply a larger weight or add a larger bias to an output result of the trained model MDL when a diagnosis subject is younger than a reference age (for example, 18 years old) for the purpose of making symptoms of the diagnosis subject estimated using the trained model MDL approximate symptoms in replies of the diagnosis subject (making a difference therebetween small). Similarly, the estimation function 122 may apply a larger weight or add a larger bias to the output result of the trained model MDL when a diagnosis subject is older than a reference age (for example, 65 years old).

A patient who has just suffered from a disease is more likely to have more fluctuations in replying to a medical examination by interview than those who do not. Therefore, the estimation function 122 in the fifth embodiment may apply a larger weight or add a larger bias to an output result of the trained model MDL when a period elapsed since a diagnosis subject suffered from a disease is shorter (not longer after the diagnosis subject suffered from the disease).

A patient who replies to a medical examination by interview for the first time at the first visit is more likely to have more fluctuations in replying to the medical examination by interview than experienced patients who have already been examined many times and replied to medical examinations by interviews. Therefore, the estimation function 122 in the fifth embodiment may apply a larger weight or add a larger bias to an output result of the trained model MDL when the diagnosis subject has less experience in replying to medical examinations by interview.

Accordingly, it is possible to remove the influence of fluctuations in replies caused by the age, onset period, reply experience, and the like of the diagnosis subject from the output result of a trained model MDL. As a result, medical staff can treat patients while further taking the uncertainty of the trained model MDL into account.

### (Sixth embodiment)

Hereinafter, a sixth embodiment will be described. The sixth embodiment differs from the above describe embodiments in that a plurality of trained models MDL are provided for ages of diagnosis subjects, a plurality of trained models MDL are provided for onset periods of diagnosis subjects, and a plurality of trained models MDL are provided for experiences of replying of diagnosis subjects in medical examinations by interview. Hereinafter, differences from the first to fifth embodiments will mainly be described, and similarities shared with the first to fifth embodiments will be omitted. In description of the sixth embodiment, parts the same as those of the first to fifth embodiments will be denoted with the same reference numerals.

For example, the learning function 126 of the sixth embodiment may generate a trained model MDL for children by using training data in which diseases that children under the age of 18 have already suffered from or diseases that the children are likely to suffer from in the future are associated with examination data of the children as correct answer labels, generate a trained model MDL for adults by using training data in which diseases that adults between the ages of 18 and 65 have already suffered from or diseases that the adjust are likely to suffer from in the future are associated with examination data of the adults as correct answer labels, and generate a trained model MDL for the elderly by using training data in which diseases that elderly people over the age of 65 have already suffered from or diseases that the elderly people are likely to suffer from in the future are associated with examination data of the elderly people as correct answer labels. Accordingly, it is possible to remove the influence of age-related reply fluctuations from the uncertainty of the trained model MDL.

For example, the learning function 126 of the sixth embodiment may generate a trained model MDL specialized for patients having onset periods of less than one year by using training data in which diseases that patients having onset periods of less than one year have already suffered from or diseases that the patients are likely to suffer from in the future are associated with examination data of the patients as correct answer labels, and generate a trained model MDL specialized for patients having onset periods of one year or longer by using training data in which diseases that patients having onset periods of one year or longer have already suffered from or diseases that the patient are likely to suffer from in the future are associated with examination data of the patients as correct answer labels. Accordingly, it is possible to remove the influence of fluctuations of replies due to the onset period from the uncertainty of the trained model MDL.

For example, the learning function 126 of the sixth embodiment may generate a trained model MDL specialized for patients having onset periods of less than one year by using training data in which diseases that patients having onset periods of less than one year have already suffered from or diseases that the patient re likely to suffer from in the future are associated with examination data of the patients as correct answer labels, and generate a trained model MDL specialized for patients having onset periods of one year or longer by using training data in which diseases that patients having onset periods of one year or longer have already suffered from or diseases that the patient are likely to suffer from in the future are associated with examination data of the patients as correct answer labels. Accordingly, it is possible to remove the influence of fluctuations of replies due to the onset period from the uncertainty of the trained model MDL.

For example, the learning function 126 of the sixth embodiment may generate a trained model MDL specialized for first-visit patients by using training data in which diseases that first-visit patients having no experience of replying to medical examinations by interview have already suffered from or diseases that the first-visit patients are likely to suffer from in the future are associated with examination data of the patients as correct answer labels, and generate a trained model MDL specialized for patients who attend second and subsequent medical examinations by using training data in which diseases that patients having one or more experiences of replying to medical examinations by interview in second and subsequent medical examinations have already suffered from or diseases that the patients are likely to suffer from in the future are associated with examination data of the patients as correct answer labels. Accordingly, it is possible to remove the influence of fluctuations of replies according to experience of medical examination by interview from the uncertainty of the trained model MDL.

### (Other embodiments)

Hereinafter, other embodiments will be described. Although the user interface 10 and the medical information processing device 100 have been described as different devices in the above-described embodiments, the present invention is not limited thereto. For example, the user interface 10 and the medical information processing device 100 may be integrated into one device. For example, the processing circuit 20 of the user interface 10 may include the estimation function 122, the determination function 123, and the learning function 126 included in the processing circuit 120 of the medical information processing device 100 in addition to the acquisition function 21, the output control function 22, and the communication control function 23. In this case, the user interface 10 can perform processing of the various flowcharts described above standalone (offline).

According to at least one embodiment described above, the processing circuit 120 of the medical information processing device 100 acquires examination data of a diagnosis subject and reply data in a medical examination by interview. The processing circuit 120 estimates a disease of the diagnosis subject by inputting the examination data of the diagnosis subject to a trained model MDL trained in advance. The processing circuit 120 further estimates symptoms of the diagnosis subject from the estimated disease of the diagnosis subject using a case database. Then, the processing circuit 120 outputs a result of comparison between the estimated symptoms of the diagnosis subject and symptoms in replies of the diagnosis subject via the output interface 113. Accordingly, medical staff can treat patients while taking the uncertainty of the trained model MDL into account.

Although several embodiments have been described, these embodiments are presented as examples and are not intended to limit the scope of the invention. These embodiments can be implemented in various other embodiments, and various omissions, replacements, and changes can be made without departing from the scope of the invention as defined by the appended claims.

## Claims

1. A medical information processing system (1) comprising:
an acquisition unit (121) configured to acquire medical examination data of a diagnosis subject and reply data of a medical examination by interview for the diagnosis subject;
an estimation unit (122) configured to estimate a disease or symptoms of the diagnosis subject by inputting the examination data of the diagnosis subject to a model (MDL) trained on the basis of a training data set in which diseases or symptoms that a learning subject suffers from are associated with examination data of the learning subject as correct answer labels; and
an output control unit (124) configured to output a result of comparison between estimated data based on an estimation result of the disease or symptoms of the diagnosis subject and the reply data via an output unit (113).

2. The medical information processing system according to claim 1,
wherein the estimation unit (122) is configured to estimate a plurality of diseases that the diagnosis subject is able to suffer from or symptoms of each of a plurality of diseases that the diagnosis subject is able to suffer from using the model, and
further comprising: a determination unit (123) configured to determine whether or not the reply data is similar to each of a plurality of pieces of estimated data corresponding to each of the plurality of diseases that the diagnosis subject is able to suffer from.

3. The medical information processing system according to claim 2,
wherein the determination unit (123) is configured to calculate a similarity between each of the plurality of pieces of estimated data and the reply data, and
wherein the output control unit (124) is configured to preferentially outputs estimated data having a higher similarity to the reply data among the plurality of pieces of estimated data via the output unit.

4. The medical information processing system according to claim 3, wherein the output control unit (124) is configured to output information representing that the higher a similarity of the estimated data is, the higher a reliability of the model is via the output unit (113).

5. The medical information processing system according to any one of claims 1 to 4, further comprising: a learning unit (126) configured to train the model such that the estimated data is more similar to the reply data.

6. The medical information processing system according to any one of claims 1 to 5,
wherein the model (MDL) is trained on the basis of the training data set in which the diseases of the learning subject are associated with the examination data of the learning subject as correct answer labels,
wherein the estimation unit (122) is configured to estimate the disease of the diagnosis subject by inputting the examination data of the diagnosis subject to the model, further estimates symptoms of the diagnosis subject from the disease of the diagnosis subject using corresponding information in which symptoms are associated with diseases, and
wherein the output control unit (124) is configured to output a result of comparison between the estimated data representing the estimated symptoms of the diagnosis subject and the reply data via the output unit (113).

7. The medical information processing system according to any one of claims 1 to 5,
wherein the model (MDL) is trained on the basis of the training data set in which symptoms of the learning subject are associated with the examination data of the learning subject as correct answer labels,
wherein the estimation unit (122) is configured to estimate symptoms of the diagnosis subject by inputting the examination data of the diagnosis subject to the model, and
wherein the output control unit (124) is configured to output a result of comparison between the estimated data representing the estimated symptoms of the diagnosis subject and the reply data via the output unit (113).

8. The medical information processing system according to any one of claims 1 to 7, wherein, when the estimated data and the reply data do not match under a precondition that the reply data is correct, the output control unit (124) outputs predetermined information via the output unit (113) to urge a medical staff member to diagnose the diagnosis subject more carefully as compared to a case in which the estimated data and the reply data match under the precondition.

9. A medical information processing system comprising:
an acquisition unit (121) configured to acquire medical examination data of a diagnosis subject and reply data of a medical examination by interview for the diagnosis subject;
an estimation unit (122) configured to estimate a disease of the diagnosis subject by inputting the examination data of the diagnosis subject to a model (MDL) trained on the basis of a training data set in which diseases that a learning subject suffers from are associated with examination data of the learning subject as correct answer labels, and further estimate symptoms of the diagnosis subject from the disease of the diagnosis subject using corresponding information in which symptoms are associated with diseases; and
an output control unit (124) configured to output a result of comparison between estimated data representing an estimation result of the symptoms of the diagnosis subject and the reply data via an output unit (113).

10. A medical information processing system comprising:
an acquisition unit (121) configured to acquire medical examination data of a diagnosis subject and reply data of a medical examination by interview for the diagnosis subject;
an estimation unit (122) configured to estimate symptoms of the diagnosis subject by inputting the examination data of the diagnosis subject to a model (MDL) trained on the basis of a training data set in which symptoms of a learning subject are associated with examination data of the learning subject as correct answer labels; and
an output control unit (124) configured to output a result of comparison between estimated data representing an estimation result of the symptoms of the diagnosis subject and the reply data via an output unit (113).

11. A medical information processing method using a computer, comprising:
acquiring medical examination data of a diagnosis subject and reply data of a medical examination by interview for the diagnosis subject;
estimating a disease or symptoms of the diagnosis subject by inputting the examination data of the diagnosis subject to a model trained on the basis of a training data set in which diseases or symptoms that a learning subject suffers from are associated with examination data of the learning subject as correct answer labels; and
outputting a result of comparison between estimated data based on an estimation result of the disease or symptoms of the diagnosis subject and the reply data via an output unit.

12. A program for causing a computer to execute:
acquiring medical examination data of a diagnosis subject and reply data of a medical examination by interview for the diagnosis subject;
estimating a disease or symptoms of the diagnosis subject by inputting the examination data of the diagnosis subject to a model trained on the basis of a training data set in which diseases or symptoms that a learning subject suffers from are associated with examination data of the learning subject as correct answer labels; and
outputting a result of comparison between estimated data based on an estimation result of the disease or symptoms of the diagnosis subject and the reply data via an output unit.
